# FASCICULE DE BREVET EUROPEEN

(11) **EP 1 036 433 B1**
(45) Date de publication et mention de la délivrance du brevet: **19.03.2003**
(21) Numéro de dépôt: 98958321.6
(22) Date de dépôt: 03.12.1998
(51) Int. Cl.: H02N 1/00, A61M 25/00

(54) **MICROACTIONNEURS ELECTROSTATIQUES, MICROCATHETERS TRIDIMENSIONNELS ACTIFS EXPLOITANT CEUX-CI ET PROCEDE DE FABRICATION**
ELEKTROSTATISCHER MICROANTRIEB, DREIDIMENSIONALER AKTIVE MIKROKATHETER UND VERFAHREN ZUR HERSTELLUNG
ELECTROSTATIC MICROACTUATORS, ACTIVE THREE-DIMENSIONAL MICROCATHETERS USING SAME AND METHOD FOR MAKING SAME

(30) Priorité: 05.12.1997 FR 9715393
(43) Date de publication de la demande: 20.09.2000
(73) Titulaire: CENTRE NATIONAL DE LA RECHERCHE SCIENTIFIQUE (CNRS), 75794 Paris Cedex 16 (FR)
(72) Inventeur: MINOTTI, Patrice, F-25660 Gennes (FR); BOURBON, Gilles, F-25000 Besançon (FR); LANGLET, Philippe, F-62149 Festubert (FR); MASUZAWA, Takahisa LIMMS/CNRS - IIS, Minato-ku Tokyo 106 (JP); FUJITA, Hiroyuki LIMMS/CNRS - IIS, Minato-ku Tokyo 106 (JP)
(74) Mandataire: Texier, Christian
(86) Numéro de dépôt international: FR9802613
(87) Numéro de publication internationale: WO99030410

(56) Documents cités:
- US-A- 5 235 225
- US-A- 5 563 466
- FUJITA H ET AL: "Group work of distributed microactuators" ROBOTICA, SEPT.-OCT. 1996, CAMBRIDGE UNIVERSITY PRESS, UK, vol. 14, pt.5, pages 487-492, XP002076440 ISSN 0263-5747
- FURUHATA T ET AL: "ARRAY-DRIVEN ULTRASONIC MICROACTUATORS ARRAYED MICROACTUATOR MODULES THAT HAVE SWING PINS" TRANSDUCERS, SAN FRANCISCO, JUNE 24 - 27, 1991, no. CONF. 6, 24 juin 1991, pages 1056-1059, XP000560535 INSTITUTE OF ELECTRICAL AND ELECTRONICS ENGINEERS
- TOSHIO FUKUDA: "GIANT MAGNETOSTRICTIVE ALLOY(GMA) APPLICATIONS TO MICRO MOBILE ROBOT AS A MICRO ACTUATOR WITHOUT POWER SUPPLY CABLES" 30 janvier 1991 , PROCEEDINGS OF THE WORKSHOP ON MICRO ELECTRO MECHANICAL SYSTEMS INVESTIGATION OF MICRO STRUCTURES, SENSORS, ACTUATORS, MACHINES AND ROBOTS, NARA, JP., JAN. 30 - FEB. 2, 1991, NR. WORKSHOP 4, PAGE(S) 210 - 215 , INSTITUTE OF ELECTRICAL AND ELECTRONICS ENGINEERS XP000295570
- PATENT ABSTRACTS OF JAPAN vol. 018, no. 012 (C-1150), 11 janvier 1994 & JP 05 253175 A (OLYMPUS OPTICAL CO LTD), 5 octobre 1993

## Description

La présente invention concerne le domaine des microactionneurs électromécaniques, c'est à dire le domaine des microsystèmes adaptés pour fournir un effort mécanique contrôlé en réponse à une excitation électrique.

Le document US-A-5235225 décrit une structure dans laquelle l'actionneur de base est constitué de deux stators parallèles munis chacun d'une pluralité d'électrodes et d'un rotor flexible, au moins partiellement électriquement conducteur disposé entre les deux stators précités. Le déplacement du rotor est opéré par une commande séquentielle, et progressant dans l'espace, d'une tension électrique appliquée sur les électrodes.

Plus précisément encore la présente invention concerne le domaine des microactionneurs électrostatiques dénommés actionneurs élémentaires distribués, «Scratch Drive Actuators » ou « SDA ».

On trouvera un descriptif de ces actionneurs dans les documents [ 1], [ 2] et [3].

Ces actionneurs proposés depuis quelques années, sont tout particulièrement destinés à l'entrainement direct des micromachines de dimensions micrométriques. Ils ont la particularité d'associer un mécanisme de transfert d'énergie mécanique par friction avec la mise en oeuvre classique d'un champ de forces électrostatiques.

On se reportera utilement aux documents précités pour bien comprendre la structure générale et le fonctionnement de ces actionneurs.

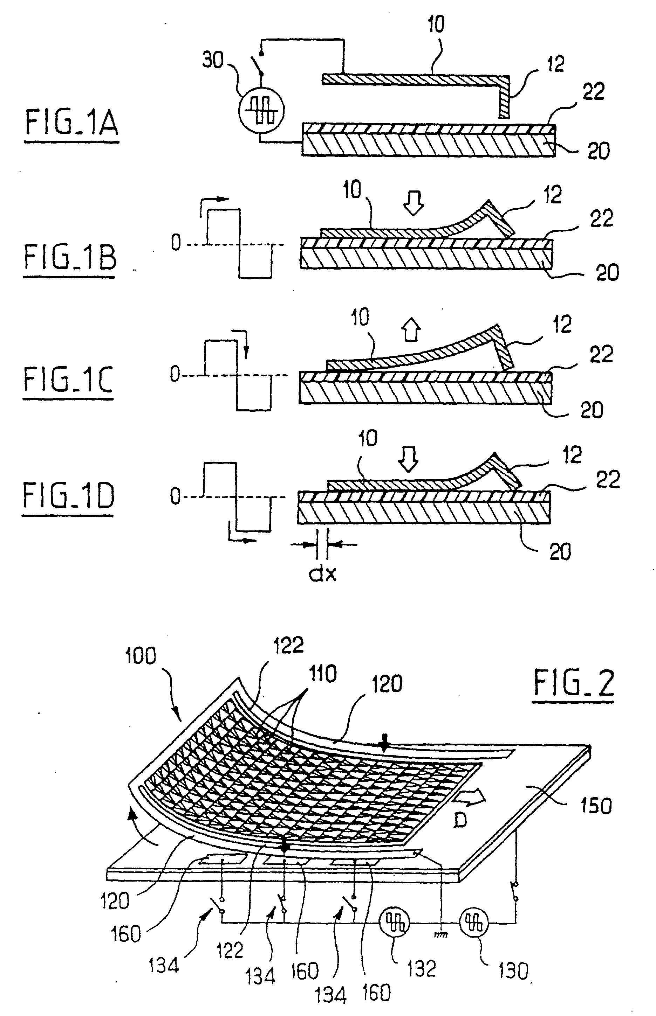

Ceux-ci sont schématisés sur les figures 1A à 1D ci-jointes.

Pour l'essentiel, un SDA comprend une plaque ou poutre 10, par exemple en polysilicium, munie à une extrémité d'un muret ou coussinet 12 en saillie, dirigé vers un substrat 20, par exemple en Silicium, revêtu d'une couche isolante 22, par exemple en Si₃N₄. Un générateur 30 est adapté pour appliquer des impulsions de tension entre la plaque 10 et le substrat 20.

Comme on le voit sur la figure 1B, sur un front montant d'impulsion, la plaque 10 est attirée vers le substrat 20 par la force électrostatique générée entre ceux-ci. L'appui du muret 12 sur la couche 22 impose une flexion statique de la plaque 10, qui entraine à son tour un déport du muret 12.

Sur le front descendant de l'impulsion, comme on le voit sur la figure 1C, la plaque 10 tend à reprendre sa géométrie de repos, grâce à l'énergie élastique emmagazinée dans la plaque 10, et se trouve alors décalée d'une amplitude dx par rapport à sa position antérieure, en raison de l'appui défini entre le muret 12 et la couche 22.

Ainsi ces systèmes permettent de convertir des oscillations mécaniques de très faible amplitude ayant pour origine la flexion statique de la plaque mince 10, en un mouvement de corps rigide de cette même plaque.

Les forces électrostatiques produites à l'interface plaque 10/substrat 20 sont d'autant plus intenses, pour une tension d'excitation donnée issue du générateur 30, que la hauteur du muret 12 située sous la plaque en flexion 10 est faible. La hauteur du muret 12 typiquement de l'ordre de un micromètre, introduit par ailleurs une forte démultiplication de vitesse dans le mécanisme de conversion d'énergie à l'interface plaque 10/substrat 20. La démultiplication de vitesse intrinsèque aux très faibles déformations mécaniques mises en jeu dans la conversion d'énergie par friction, concourt à une multiplication duale des forces motrices générées au cours du déplacement de la plaque. Les SDA ont ainsi la particularité de développer des forces utiles importantes à basse vitesse, en l'absence d'une quelconque réduction de vitesse auxiliaire.

La longueur du pas de déplacement dépend de la hauteur du muret 12, de la raideur de la plaque 10 et de la tension de commande appliquée. Le pas de déplacement est typiquement de l'ordre de 25 nanomètres pour une plaque 10 présentant une largeur de l'ordre de 50 micromètres, une épaisseur de l'ordre de 1 micromètre et une longueur de l'ordre de 60 micromètres.

La répétition de tels cycles permet d'accumuler des pas de déplacement et par conséquent permet un déplacement relatif important entre la plaque 10 et le substrat 20.

Cependant bien que se montrant très prometteurs, à la connaissance des inventeurs, les SDA sont jusqu'ici restés à l'échelle du laboratoire et n'ont pas connu de développement industriel.

Cela semble du en particulier au fait que l'effort généré par les SDA connus reste limité même si celui-ci est important à l'échelle micrométrique. Cet effort, typiquement de l'ordre de 50 à 100 micro-newton pour un SDA alimenté à une tension crête d'excitation de l'ordre de 100V, ne peut satisfaire qu'un nombre limité d'applications exclusivement réservées à l'échelle des micromachines.

Et les tentatives d'accroitre notablement cet effort en augmentant la taille des SDA n'ont pas été couronnées de succès jusqu'ici.

En effet d'une part les forces électrostatiques mises en jeu dans l'actionnement décroissent très rapidement avec l'augmentation des dimensions des SDA. D'autre part les procédés d'élaboration mis en jeu dans la fabrication des SDA interdisent l'élaboration de dispositifs ayant une épaisseur supérieure à quelques microns, ce qui constitue une limitation intrinsèque à l'augmentation des autres dimensions du SDA.

La présente invention a maintenant pour but de proposer de nouveaux moyens permettant de mettre en oeuvre industriellement des SDA.

Allant à l'encontre des tentatives actuelles tendant à augmenter la taille d'un SDA pour obtenir un effort de sortie acceptable, dans le cadre de la présente invention il est proposé de conserver des SDA de taille réduite, mais de multiplier ceux-ci et de les associer dans des conditions adéquates pour permettre une addition des forces générées par chacun de ces SDA, à savoir en utilisant des moyens adaptés pour, d'une part appliquer sur lesdits SDA une précontrainte mécanique extérieure propre à permettre une superposition des forces générées par les différents SDA, et d'autre part, communiquer à une charge extérieure la totalité de la force motrice issue du comportement collectif de ces mêmes SDA.

Cette précontrainte mécanique des SDA est avantageusement obtenue à l'aide d'une tension de polarisation appliquée au repos à l'ensemble des SDA.

Pour permettre de communiquer à une charge extérieure la totalité de la force motrice issue du comportement collectif des SDA, selon une caractéristique avantageuse de la présente invention, la feuille portant ces derniers est placée dans un jeu mécanique à l'interface de deux corps solides articulés entre eux.

Les inventeurs ont en effet démontré qu'une telle précontrainte, associée à des moyens garantissant la communication de la force motrice, à la charge extérieure, était indispensable pour permettre un cumul des efforts générés par les différents SDA.

La coopération de microactionneurs a déjà été exploitée dans le domaine de la motorisation de micromachines mais, à la connaissance des inventeurs, uniquement au travers du développement de convoyeurs d'objets par friction dans le plan horizontal, afin de profiter de la gravité de l'objet déplacé. Pour de tels dispositifs, les forces motrices communiquées à l'élément mobile ne dépendent que de la masse de l'objet déplacé, ainsi que du coefficient de friction à l'interface objet/actionneurs (conformément aux lois de Coulomb sur le frottement solide). Dans ce cas les forces motrices communiquées à l'objet déplacé sont indifférentes tant du nombre que des caractéristiques motrices des actionneurs participant à la motorisation. De plus, ces mêmes forces motrices dépendent de la configuration de la machine (ou convoyeur), dans l'espace et notamment de l'horizontalité du plan de transfert de l'objet. Il est clair, par conséquent, que la multiplication du nombre d'actionneurs participant à la motorisation d'une seule et même charge, ne conduit pas nécessairement à une multiplication homologue des forces utiles mises en jeu dans la motorisation.

La présente invention se distingue des inventions précédentes parce qu'elle n'utilise pas la gravité (ou toute autre solution telle que la déformation élastique d'un ressort d'appui...), pour calibrer une précontrainte dans le processus de transmission de puissance mécanique. Elle utilise des forces d'attraction électrostatiques particulièrement intenses compte tenu de l'échelle des SDA, pour calibrer une précontrainte individualisée sur chaque actionneur impliqué dans la collectivité. Cette précontrainte est intrinsèque à chaque actionneur dans la mesure où elle est indifférente aux paramètres de la charge déplacée, contrairement aux dispositifs de l'art antérieur qui mettent en jeu la gravité de manière systématique. L'application de la précontrainte, dans le cadre de la présente invention, est par ailleurs naturelle car l'attraction électrostatique ne nécessite pas le recours à la déformation élastique d'un ressort d'appui auxiliaire. Pratiquement, celle-ci est obtenue à l'aide d'une tension de polarisation appliquée au repos à l'ensemble des SDA, comme indiqué précédemment. Par ailleurs, les forces électrostatiques ne dépendent que de la position relative du SDA sur son substrat et sont indifférentes de la situation du substrat dans l'espace tridimensionnel.

L'invention proposée garantit par conséquent, contrairement aux dispositifs collectifs de l'art antérieur, une superposition effective des forces individuelles de chaque SDA, quelle que soit par ailleurs la configuration spatiale de la colonie considérée.

Les inventeurs ont par ailleurs constaté que les treillis support jusqu'ici proposés, et susceptibles d'associer plusieurs SDA, s'avèrent incapables de conserver leur intégrité mécanique lors de la transmission de forces extérieures importantes. Ces treillis connus sont formés généralement d'un assemblage de poutres extrêmement fragiles, ayant comme les SDA, une épaisseur de l'ordre de un micromètre (celles-ci étant fabriquées au même moment que les SDA, au cours d'un même processus et avec un matériau similaire). La fragilité des treillis connus est donc une limitation intrinsèque à la transmission d'efforts importants.

Ainsi selon une autre caractéristique avantageuse de la présente invention, allant à l'encontre des tentatives actuelles, les inventeurs proposent d'insérer des feuilles flexibles, par exemple en polysilicium, comportant un grand nombre de SDA, dans un jeu mécanique séparant deux corps solides articulés l'un par rapport à l'autre. Cette solution technique permet en effet de faire coopérer pratiquement un très grand nombre de SDA, dans des conditions propices à une superposition effective des forces motrices et de telle sorte que la transmission de puissance mécanique, qui résulte de l'accumulation des forces utiles produites par la collectivité, ne puisse être répercutée sur la structure matérielle raccordant l'ensemble des SDA.

A cette fin dans le cadre de la présente invention, les feuilles de SDA sont avantageusement constituées d'un cadre, en contact uniquement avec l'un des corps solides impliqués dans l'articulation, par exemple le bâti. Les SDA ne sont quant à eux qu'au contact exclusif de l'autre corps solide impliqué dans l'articulation, par exemple l'arbre moteur. Une telle configuration autorise une accumulation de force proportionnelle au nombre de SDA impliqués à l'interface. Elle garantit aussi et surtout l'intégrité du cadre (parce que celui-ci est renforcé au contact du bâti), quelle que soit la transmission de puissance mécanique extérieure communiquée à l'élément mobile (ou arbre moteur).

L'invention concourt ainsi, dans sa globalité, à la possibilité physique et matérielle (ou mécanique) d'impliquer un très grand nombre de SDA dans l'entraînement d'une seule et même charge, contrairement aux solutions connues de l'art antérieur.

Typiquement un système conforme à la présente invention intègre ainsi de quelques dizaines à quelques milliers de SDA.

Par ailleurs la présente invention propose des moyens spécifiques de mise en forme d'une feuille, par exemple en polysilicium, comportant un grand nombre de SDA, par flexion de barres venues de cette feuille.

La présente invention concerne également un procédé de mise en forme d'une feuille comportant un grand nombre de SDA, exploitant de tels moyens.

Selon une autre caractéristique avantageuse de la présente invention, le système peut également comprendre des moyens formant capteur de force, par exemple capteur de couple, comportant au moins une poutre intégrée à la feuille formant les SDA et adaptée pour être déformée lors de l'actionnement du système, ladite poutre étant associée à des moyens d'analyse de sa déformation.

D'autres buts et avantages de la présente invention apparaitront à la lecture de la description détaillée qui va suivre et en regard des dessins annexés, donnés à titre d'exemples non limitatifs et sur lesquels :
- les figures 1A à 1D précédemment décrites illustrent schématiquement la structure générale et le fonctionnement d'un SDA classique,
- la figure 2 illustre schématiquement un processus de décollement automatique d'une feuille de SDA par rapport à son substrat support, conforme à la présente invention,
- la figure 3 schématise un processus d'enroulement d'une feuille de SDA conforme à la présente invention, autour d'un arbre moteur tubulaire,
- la figure 4 schématise une architecture mécanique d'une cellule élémentaire de SDA conforme à la présente invention,
- la figure 5 schématise une feuille de SDA conforme à la présente invention, positionnée au niveau d'une interface entre un rotor et un bâti,
- les figures 6, 7 et 8 représentent respectivement un moteur annulaire conforme à la présente invention, une vue partielle en coupe transversale de celui-ci, associé à ses moyens de commande électrique et une schématisation d'un capteur de couple intégré à ce moteur annulaire,
- la figure 9 représente une vue schématique en perspective d'un cathéter conforme à la présente invention, mettant en oeuvre une pluralité d'actionneurs SDA du type précité,
- la figure 10 représente une vue en coupe longitudinale du même cathéter,
- la figure 11 représente un module de base d'un tel cathéter,
- la figure 12 illustre la déformation d'un tel module sous l'effet d'un actionneur à base d'un matériau à mémoire de forme, et
- la figure 13 schématise l'utilisation d'un tel cathéter conforme à la présente invention dans un vaisseau sanguin.

La structure générale connue des SDA ne sera pas décrite dans le détail par la suite.

Il en est de même de la technologie de fabrication de ceux-ci. Cette technologie dérivée de la technologie de fabrication des circuits intégrés est en effet connue de l'homme de l'art.

Cependant on décrira par la suite un exemple de procédé de fabrication d'une feuille de SDA conforme à la présente invention.

Les dimensions nominales d'un SDA sont typiquement de l'ordre de quelques dizaines de micromètres de côté. Dans le cadre de la présente invention on peut ainsi prévoir des centaines, voire des milliers de SDA juxtaposés sur une surface de l'ordre du mm².

De préférence dans le cadre de la présente invention, les SDA sont réalisés par usinage chimique de feuilles minces en polysilicium dopé. Leur configuration peut faire l'objet de nombreuses variantes de réalisation. Il en est de même des conditions de raccordement des SDA à la trame de la feuille, de la discrétisation de la feuille en cellules élémentaires (nombre de SDA par unité de surface) et de la surface de la feuille.

Comme on l'a indiqué précédemment dans le cadre de la présente invention, pour permettre une addition des efforts élémentaires générés par chaque SDA, ceux-ci sont soumis à une précontrainte mécanique extérieure, de préférence sous forme d'une tension de polarisation au repos, qui impose une flexion contrôlée permanente à la plaque 10 de chaque SDA.

Comme on l'a indiqué précédemment, dans le cadre de la présente invention, il est prévu en outre des moyens adaptés pour assurer que la totalité de la force motrice issue du comportement collectif des SDA soit communiquée à la charge extérieure.

Pour ce faire la feuille de SDA est de préférence placée dans le jeu mécanique entre deux corps solides articulées entre eux.

La feuille de SDA peut ainsi être placée entre le substrat sur lequel ladite feuille de SDA a été réalisée, et un corps solide rapporté.

Cependant dans le cadre de la présente invention, la feuille de SDA est de préférence séparée tout d'abord de son substrat support, puis insérée dans le jeu mécanique précité existant entre deux corps solides articulés entre eux.

Les feuilles en polysilicium comportant une colonie de SDA, conformes à la présente invention, présentent une grande flexibilité dès qu'elles sont décollées du substrat de silicium sur lequel elles ont été chimiquement usinées.

Ont peut donc prévoir différentes solutions pour transférer ces feuilles sur leur site d'utilisation.

Selon une mise en oeuvre particulièrement avantageuse de la présente invention, illustrée sur la figure 2, le décollement de la feuille comportant les SDA, par rapport à son substrat support, est opéré grâce à des barres de flexion réparties à la périphérie de la feuille.

Bien entendu en variante on peut prévoir de telles barres de flexion en un autre lieu de la feuille, par exemple dans la masse de celle-ci, et non pas seulement à sa périphérie.

On aperçoit ainsi sur la figure 2 une feuille 100 de polysilicium comportant un grand nombre de SDA élémentaires 110. Cette feuille 100 repose sur le substrat en silicium 150 sur lequel ladite feuille 100 a été usinée.

Cette feuille 100 comporte deux barres de flexion 120 parallèles disposées respectivement le long de deux bords opposés de la feuille 100. Les barres de flexion 120 sont séparées sur leur longueur, de la masse de la feuille 100 grâce à des découpes longitudinales 122 formées entre lesdites barres 120 et le corps central de la feuille 100. Les barres de flexion 120 sont ainsi usinées à partir de la masse de la feuille 100, Cependant les barres de flexion 120 restent solidaires, par l'une de leurs extrémités, de ce corps central de la feuille 100.

Lorsque les premiers rangs de SDA, transversaux aux bords latéraux précités et aux barres de flexion 120 ainsi que opposés aux zones de raccordement desdites barres 120 sur la feuille 100, sont activés, la feuille 100 avance dans le sens illustré par la flèche référencée D sur la figure 2. Pour cela une tension peut être appliquée entre les plaques 10 des SDA 110 concernés et une électrode enterrée dans le substrat 150.

Ainsi si les barres de flexion 120 sont par contre maintenues en position par rapport au substrat 150 au moins sur une partie de leur longueur, le déplacement de la feuille 100 entraine une flexion des barres 120 dans une direction orthogonale au plan du substrat 150.

Et comme on le voit sur la figure 2, cette flexion des barres 120 provoque le décollement de la feuille 100 par rapport au substrat 150.

Le maintien des barres 120 par rapport au substrat 150 peut être obtenu par de nombreux moyens. De préférence dans le cadre de la présente invention ce maintien est obtenu lui même grâce à des forces électrostatiques. Pour cela de préférence, comme on le voit sur la figure 2, il est prévu des électrodes spécifiques 160 sur le substrat 150, en regard des barres 120. L'application d'une tension d'excitation entre ces électrodes 160 et les barres 120, à l'aide d'un générateur 132, permet ainsi de plaquer les barres 120 contre les électrodes 160.

Plus précisément encore, selon le mode de réalisation préférentiel illustré sur la figure 2 annexée, il est prévu plusieurs électrodes 160 discrètes réparties en regard des barres 120. Et chacune de ces électrodes 160 peut être alimentée sélectivement par le générateur 132, par l'intermédiaire d'un jeu d'interrupteurs respectifs 134. Ainsi la fermeture sélective d'un choisi ou de plusieurs interrupteurs 134, permet de sélectionner la ou les électrodes 160 alimentée(s) et par conséquent de contrôler la zone des barres 120 maintenue sur le substrat 150.

Les inventeurs ont montré qu'avec cette technique des rayons de courbure de quelques dizaines de microns peuvent être obtenus sur des feuilles de polysilicium 100.

La feuille 100 comportant les SDA 110 peut ainsi être reportée sur tout site d'utilisation souhaitée.

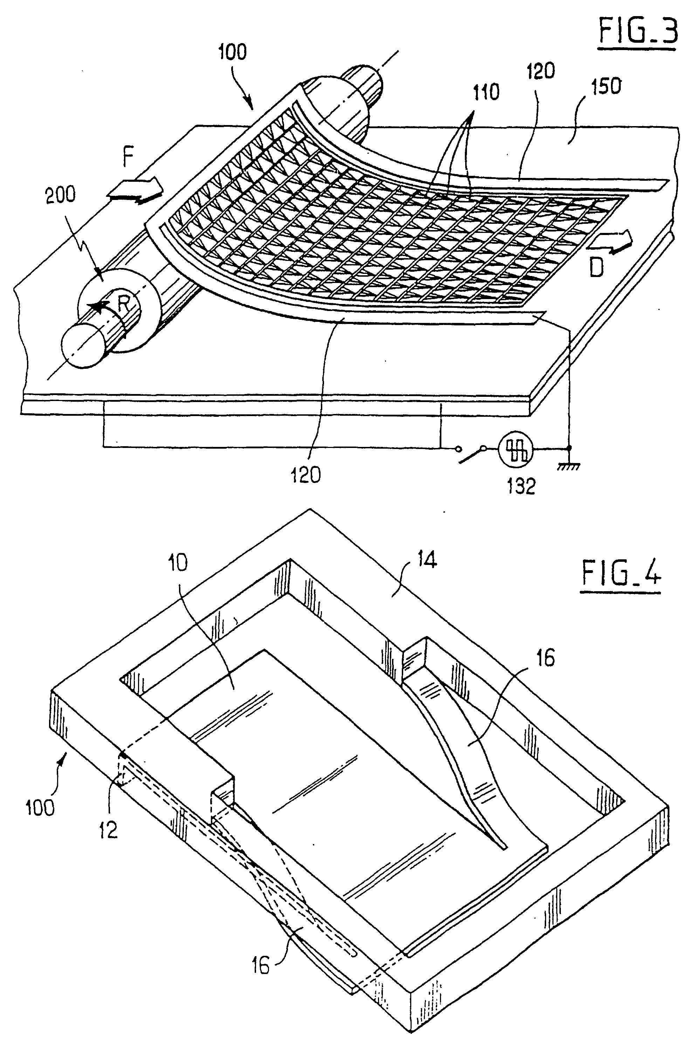

On a illustré sur la figure 3 le report d'une feuille 100 sur un arbre moteur 200.

Cet arbre 200 peut lui même faire l'objet de nombreux modes de réalisation. Selon le mode de réalisation particulier et non limitatif illustré sur la figure 3, il s'agit d'un arbre tubulaire dont l'enveloppe extérieure est cylindrique de révolution.

Pour assurer le transfert de la feuille 100 sur l'arbre 200, celui-ci est déposé sur le substrat 150 au niveau de l'extrémité décollée de la feuille 100. Puis l'arbre 200 est déplacé sous la feuille 100 au fur et à mesure du décollement de celle-ci, comme illustré par la flèche illustrée F sur la figure 3. Simultanément de préférence l'arbre 200 est animé d'un mouvement de rotation autour de son axe (comme schématisé par la flèche R sur la figure 3) de sorte que l'arbre 200 vient soutenir la feuille 100 au fur et à mesure de son décollement, sans déplacement relatif entre la surface de l'arbre 200 et la feuille 100. Ainsi la feuille est progressivement transferrée, sans risque de dommage, sur l'arbre 200.

L'enroulement de la feuille 100 sur l'arbre 200 est facilité si l'on exploite simultanément une attraction électrostatique vers l'arbre moteur 200 lui même.

Pour cela la feuille de polysilicium 100 peut être soumise à un potentiel de l'ordre de 100 à 200 V crête, tandis que l'arbre moteur est mis à la masse (ou inversement). Bien entendu une couche électriquement isolante doit être prévue entre la feuille 100 et l'arbre 200. A cette fin l'arbre 200 peut être réalisé par exemple à partir d'un matériau électriquement conducteur oxydé en surface.

La présente invention n'est aucunement limitée à la réalisation d'un moteur tubulaire comme décrit en regard de la figure 3. Elle peut trouver application dans un grand nombre de configurations, telles que par exemple dans des moteurs linéaires.

Par ailleurs selon l'orientation donnée aux SDA, sur les lignes et les colonnes d'une même feuille, il est possible de réaliser des moteurs à plusieurs degrés de liberté.

Ainsi par exemple en exploitant deux séries de SDA possédant des orientations orthogonales, on peut réaliser des translateurs plans de type X-Y. On peut également réaliser des moteurs de type verrou associant un degré de liberté de rotation à un degré de liberté de translation.

L'insertion de la feuille 100 comportant les SDA 110 à l'interface entre le rotor 200 et un bâti externe associé, peut être réalisée de diverses façons.

De préférence dans une première phase, la feuille 100 est soumise à un potentiel de l'ordre de 100V tandis que le rotor 200 est mis à la masse. La feuille 100 est donc solidaire de l'arbre moteur 200, ce qui lui confère la rigidité nécessaire pour résister ultérieurement aux efforts d'insertion de l'arbre moteur dans le bâti.

L'insertion de l'arbre moteur dans son logement est ensuite opérée.

Puis dans une phase ultérieure, la feuille 100 est bloquée par rapport au logement précité afin d'autoriser le mouvement relatif du rotor 200 par rapport au bâti. Pour satisfaire ce blocage le bâti du moteur est à son tour mis à la masse dans le but d'attirer le cadre de la feuille 100 de SDA.

Le blocage en rotation de la feuille 100 est donc garanti par un champ de forces électrostatiques à l'interface feuille 100/bâti, ainsi que par les frottements secs induits par le contact de la feuille 100 sur le bâti. La feuille 100 doit être dimensionnée (en épaisseur notamment) de telle sorte que le cadre de la feuille 100 soit naturellement attiré par le bâti, tandis que les SDA 110 restent attirés par le rotor 200.

Pour cela chaque plaque 10 formant un SDA est supporté élastiquement dans un cadre 14 ménagé dans la feuille 100. Par exemple comme illustré sur la figure 4, chaque plaque 10 formant un SDA est supporté par un cadre 14 ménagé dans la feuille 100 par l'intermédiaire de deux barres de suspension parallèles 16. Celles-ci relient un bord longitudinal respectif de la plaque 10 opposé au muret 12, et le cadre 14. Les barres de suspension s'étendent perpendiculairement à la direction du muret 12.

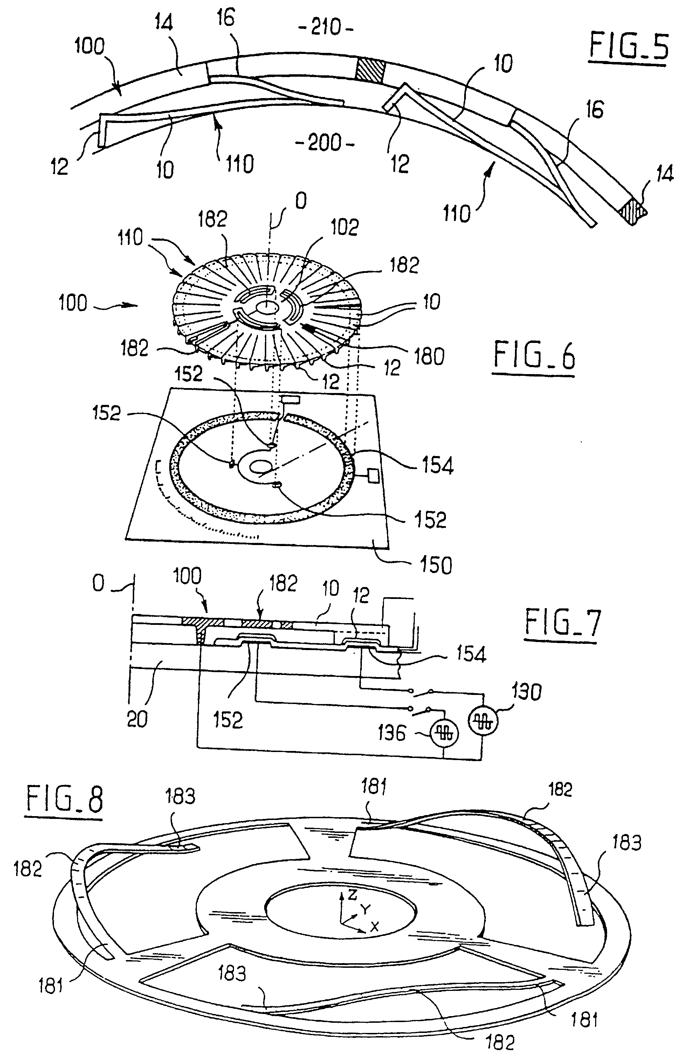

Sur la figure 5 le bâti du moteur qui entoure le rotor 200 est référencé 210. Là encore une couche électriquement isolante doit être prévue entre le bâti 210 électriquement conducteur et les cadres 14. Cette couche isolante peut être obtenue par oxydation de la surface interne du bâti 210.

Une fois qu'elle a été ainsi fixée sur le bâti 210, la feuille 100 joue le rôle de stator.

Par ailleurs les barres de suspension précitées 16 sont de préférence adaptées pour permettre un déplacement radial important des SDA vis à vis du cadre 14 et permettre ainsi de compenser l'incertitude portant sur le jeu mécanique inévitable à l'interface rotor 200/bâti 210.

En effet les tolérances de fabrication mécaniques normalisées des dispositifs macroscopiques garantissent au mieux un jeu mécanique avec une incertitude de l'ordre d'une dizaine de microns pour un accouplement de 1mm de diamètre, soit une incertitude largement supérieure à deux fois l'épaisseur de la feuille de SDA 100.

Les barres 16 de suspension permettent ainsi une accomodation radiale de la feuille 100 dans le bâti 210, de sorte que les cadres 14 soient bloqués contre le bâti 210, tandis que les SDA sont en appui sur le rotor 200.

Les applications potentielles de la présente invention sont nombreuses. Elles concernent notamment la motorisation des micromachines depuis l'échelle micrométrique, jusqu'à l'échelle millimétrique.

On notera que les feuilles de SDA 100 peuvent s'insérer naturellement dans des liaison mécaniques surfaciques présentant un jeu de fonctionnement.

Ainsi les feuilles 100 de SDA occupent un volume quasi nul du point de vue de l'encombrement effectif. La présente invention permet par conséquent de réaliser des moteurs présentant un facteur volume /puissance, bien supérieur à tout système existant.

Par ailleurs dans la mesure ou les feuilles de SDA occupent un volume effectif nul, il est possible de les intégrer aisément dans des liaisons mécaniques d'un mécanisme sans affecter l'architecture mécanique de celui-ci.

De même les feuilles 100 de SDA conformes à la présente invention peuvent être intégrées dans des architectures mécaniques d'une articulation traditionnelle, sans encombrement additionnel, et ainsi donner lieu à l'infrastructure d'un moteur. Les liaisons mécaniques produisent alors de l'énergie mécanique, alors qu'elles en dissipent traditionnellement depuis toujours en raison du frottement sec à l'interface des corps solides.

On a représenté sur les figures 6 à 8 une variante de réalisation selon laquelle le moteur est de type annulaire, c'est à dire qu'il comporte une feuille plane 100 comportant un grand nombre de SDA 110, en forme de disque transversal à son axe de rotation 0.

Un tel moteur est ainsi de type bidimensionnel par opposition au moteur précédemment décrit de type tridimensionnel.

La feuille 100 qui constitue un rotor comporte un grand nombre de SDA 110 agencés radialement à partir d'un moyeu central 102.

Typiquement mais non limitativement, le rotor 100 peut ainsi comprendre 36 SDA équirépartis autour de l'axe de rotation O. Les murets 12 de chacune des plaques 10 en forme de secteur de couronne, s'étendent le long d'un bord longitudinal de ces plaques, selon un rayon dressé à partir de l'axe de rotation O, et de préférence sur une partie seulement de l'extension radiale de ces secteurs de couronne, comme on peut le voir notamment sur les figures 6 et 7.

La rotation du rotor autour de l'axe O est obtenue grâce à une suite de déformation des plaques 10, conformément au principe précédemment décrit en regard des figures 1, grâce à des impulsions appliquées par un générateur 130 entre les plaques 10 et une électrode 154 intégrée au support 150 formant stator sur lequel est disposé le rotor.

En outre le moteur illustré sur les figures 6 à 8 comprend un capteur de couple de rotation 180. Celui-ci est agencé au centre de la feuille 100.

Ce capteur 180 comprend au moins une poutre incurvée 182, centrée autour de l'axe de rotation O, dont une extrémité 181 est solidaire de la feuille 100, tandis que l'autre extrémité 183 de la poutre 182 est libre par rapport à ladite feuille 100.

Selon le mode de réalisation illustré sur la figure 6, le capteur 180 comporte trois poutres 182 équiréparties autour de l'axe de rotation O du rotor.

Les poutres 182 s'étendent dans le sens de rotation à partir de l'extrémité 181 liée à la feuille 100.

Elles sont de préférence formées par usinage chimique dans la masse de la feuille 100 et de forme générale circulaire, en secteur de couronne.

Lorsque aucun effort n'est exercé sur les poutres 182, celles-ci sont contenues dans le plan de la feuille 100 et par conséquent aucun couple externe freinant n'est appliqué sur le rotor 100.

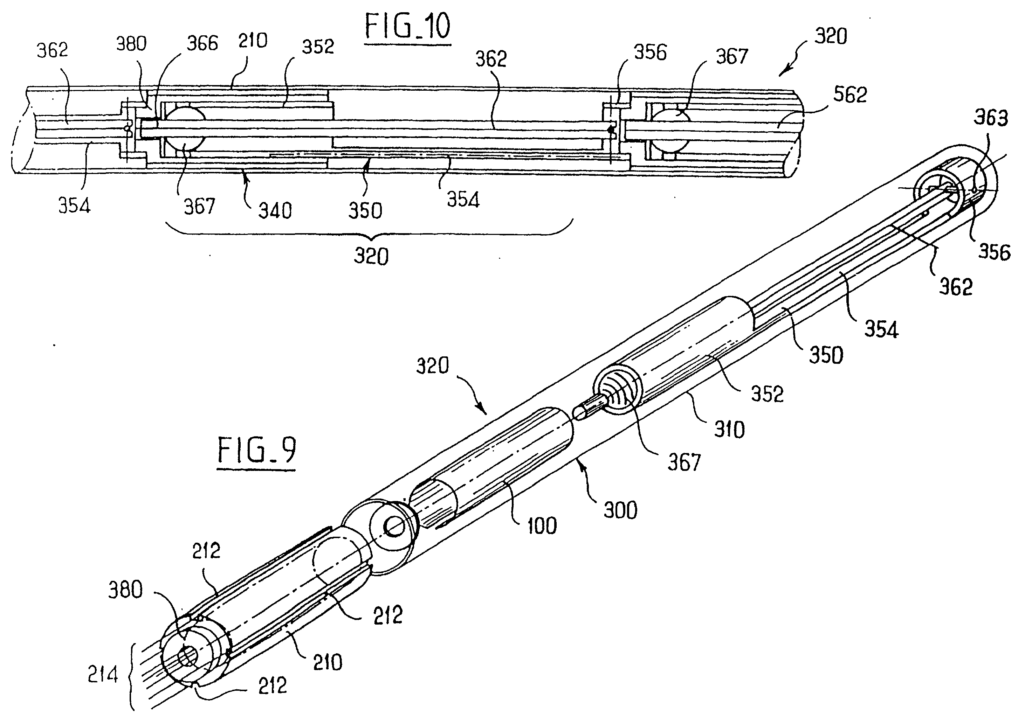

Cependant si l'extrémité libre 183 des poutres 182 est plaquée contre le support 150 du rotor qui forme stator, les poutres 182 sont soumises à un effort qui provoque leur flexion, comme schématisé sur la figure 8. Et l'amplitude de cette flexion dépend directement du couple moteur généré par les SDA. Ainsi la mesure de l'amplitude de la déformation des poutres 182 permet de mesurer directement le couple moteur des SDA.

Les poutres 182 peuvent ainsi être plaquées sur le support 150 par des forces électrostatiques résultant de l'application d'une tension adéquate appliquée par un générateur 136 entre chaque poutre 182 et des électrodes 152 formées sur le support 150 en regard du trajet de déplacement des poutres 182.

Sur la figure 6, on a illustré sous la référence 154 une électrode annulaire formée sur le support 150, en regard du trajet de déplacement des murets 12. Cette électrode 154 sert d'électrode d'entrainement et permet l'application d'une tension d'excitation entre cette électrode 154 et les plaques 10 des SDA, à l'aide du générateur 130.

La structure illustrée sur la figure 6 permet la réalisation de moteurs présentant par exemple un diamètre de l'ordre de 500 micromètres et une hauteur totale de quelques micromètres,

On va maintenant décrire un exemple de procédé de fabrication d'une feuille de SDA conforme à la présente invention.

Une pastille de silicium de type n à 20 Ω cm est préparé par nettoyage dans une solution d'acide fluoridrique tamponnée. Après rinçage à l'eau et séchage sous N_{2,} la pastille est oxydée thermiquement à 1100°C sous O₂. Une couche d'oxyde de silicium d'une épaisseur de l'ordre de 0,35 micromètre est ainsi formée à la surface de la pastille. Une couche de polysilicium d'une épaisseur de l'ordre de 0,5 micromètre d'épaisseur est ensuite déposée à 600°C par LPCVD (dépôt vapeur basse pression). Afin de réduire la résistivité du polysilicium, du phosphore est implanté selon un dosage à 5 x 10 ¹⁵cm⁻² sous une tension d'accélération de l'ordre de 50keV. Après une première lithographie, la couche de polysilicium est gravée par plasma SF₆ de manière à obtenir un écran. Une couche de nitrure de silicium riche en silicium, d'une épaisseur de l'ordre de 0,3 micromètre, est déposée ensuite sur toute la surface de la pastille à 800°C par LPCVD. Cette couche protège la couche d'oxyde de silicium à l'égard d'une gravure à l'acide fluoridrique au cours d'une étape de gravure sacrificielle finale.

Puis une couche d'oxyde de silicium d'une épaisseur de l'ordre de 2 micromètres d'épaisseur est déposée en tant que matériau sacrificiel à 600°C par LPCVD.

Après une seconde lithographie, des moules de murets sont conformés par gravure ionique réactive (RIE) avec un plasma CHF₃ + O₂. La profondeur des moules de muret préalablement réalisés, détermine bien entendu la hauteur des murets qui seront formés. Par contrôle du temps de gravure, une profondeur de muret de l'ordre de 1,5 micromètre peut être obtenue. Ainsi une couche de l'ordre de 0,5 micromètre est préservée entre la couche de nitrure et les murets.

Une troisième lithographie destinée à la réalisation dès contacts est ensuite mise en oeuvre. L'oxyde de silicium LPCVD et le nitrure de silicium sont retirés par RIE sous plasma CHF₃ + O_{2,} de sorte que une couche de polysilicium déposée par la suite puisse contacter localement la couche d'écran enterrée.

Une couche de polysilicium d'une épaisseur de l'ordre de 1,0 micromètre destinée à être ensuite conformée comme composant principal est alors déposée sur la surface de la pastille à 600°C par LPCVD. L'épaisseur de SDA est déterminée par l'épaisseur de polysilicium. La couche de polysilicium structurelle est dopée par implantation de phosphore (5 x 10 ¹⁵ cm⁻² sous une tension d'accélération de l'ordre de 150keV) et est ensuite conformée par RIE sous plasma SF₆ + SiCl₄ au cours d'une quatrième étape de lithographie.

Afin de relacher les contraintes résiduelles dans le polysilicium, la pastille est chauffée dans une atmosphère neutre de N₂ à 1100°C pendant 60mn, après dépôt d'une couche fine d'oxyde de silicium par LPCVD. Cette couche d'oxyde de silicium sert à protéger la surface de polysilicium vis à vis de l'azote. Simultanément on opère une diffusion et une activation de phosphore dans le polysilicium.

Finalement la pastille est plongée dans un bain de HF à 50% afin de dissoudre totalement l'oxyde de silicium sacrificiel.

Puis la pastille est rincée dans une solution adéquate (par exemple eau + alcool isopropyl) et séchée sous atmosphére neutre (par exemple azote).

Le microactionneur à base de SDA précédemment décrit peut trouver application dans un grand nombre de domaines.

On va maintenant décrire par la suite un exemple non limitatif d'application de ce microactionneur, dans la réalisation d'un cathéter, en regard des figures 9 à 13 annexées.

On a ainsi illustré sur les figures 9 à 13 annexées un cathéter actif 300 de très petit diamètre (typiquement de l'ordre de 1mm), qui comprend dans un tube souple d'enveloppe 310, une série de modules 320 juxtaposés sur la longueur du tube 310.

Le tube 310 peut être formé par exemple en matériau polymère.

Chaque module 320 comporte un moteur électrostatique tubulaire 340, un rotor élastique 350 et un actionneur à mémoire de forme 360.

Le moteur électrostatique 340 peut être globalement conforme aux dispositions précédemment décrite en regard de la figure 5. Ainsi le moteur 340 comporte de préférence une feuille 100 de SDA placée dans le jeu mécanique entre le rotor 350 (qui correspond à l'élément 200 de la figure 5) et un bâti tubulaire 210.

Le bâti tubulaire 210 est muni de gorges longitudinales 212 sur sa périphérie extérieure, pour permettre le passage de fils d'alimentation électrique isolés 214, nécessaires à l'alimentation des modules 320 situés en aval.

Le rotor 350 peut faire l'objet de nombreux modes de réalisation. Selon le mode de réalisation non limitatif représenté sur les figures 9 à 13, le rotor 350 comprend deux tronçons tubulaires 352, 356 d'extrémité reliés entre eux par un bras de liaison longitudinal 354. Les deux tronçons tubulaires 352 et 356 ont des diamètres identiques. Le bras de liaison 354 est de préférence formé par usinage d'un tube définissant à ses extrémités lesdits tronçons 352 et 356. Ainsi le bras de liaison 354 est de préférence constitué d'une barre longitudinale rectiligne au repos, en forme de secteur de cylindre dont le rayon de courbure correspondant à celui des tronçons 352 et 354. Le tronçon 352 du rotor a une longueur sensiblement identique à la longueur du bâti 210, et ledit tronçon 352 est introduit dans ce bâti 210, de sorte que le bras de liaison 354 et le second tronçon 356 émergent à l'extérieur du bâti 210.

Le rotor 350 au moins au niveau du tronçon 352 doit être réalisé en matériau électriquement conducteur, pour permettre l'application de forces électrostatiques sur la feuille de SDA 100. Cependant la surface externe du tronçon 352 doit être électriquement isolante, par exemple par oxydation.

Comme indiqué précédemment le rotor 350 doit être suffisamment souple pour accepter des flexions sous l'effet de la commande de l'actionneur à mémoire de forme 360.

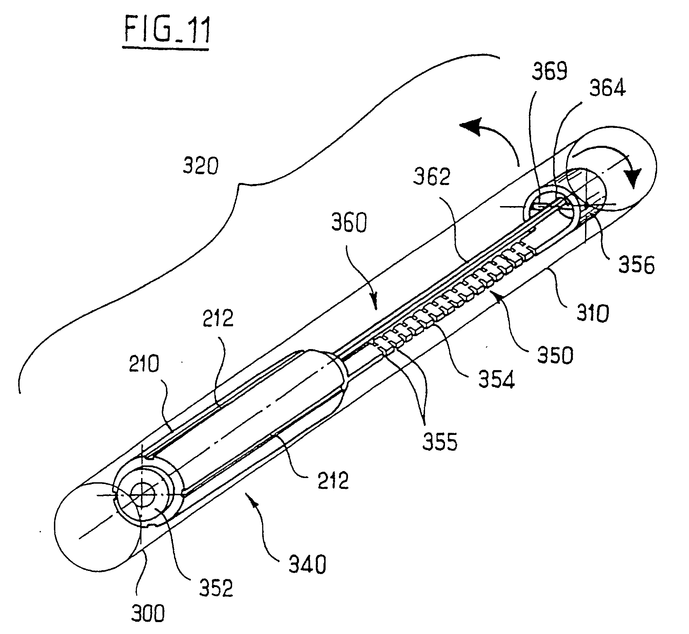

Le cas échéant comme on le voit sur la figure 11, le bras de liaison 354 peut être muni d'une série d'échancrures transversales 355 réparties sur sa longueur, entre les deux tronçons 352 et 356, et débouchant sur ses deux bords longitudinaux, ou de tous moyens équivalents, pour obtenir la souplesse adéquate.

L'actionneur à mémoire de forme 360 peut également faire l'objet de nombreux modes de réalisation. Selon le mode de réalisation préférentiel illustré sur les figures annexées, l'actionneur 360 est formé d'un barreau 362 centré sur le rotor 350 et dont les extrémités 364, 366 sont engagées respectivement dans les tronçons 356 et 352 du rotor.

Le barreau 362. peut être formé par exemple en NiTi.

L'une des extrémités du barreau 362 est fixée sur l'un des tronçons du rotor, tandis que l'autre extrémité du barreau 362 est de préférence adaptée à l'autre tronçon du rotor, avec liberté de déplacement longitudinal par rapport à celui-ci, pour autoriser une flexion du barreau 362 et du rotor 350.

A titre d'exemple, comme on l'a illustré sur les figures 9 à 13, l'extrémité 364 du barreau 362 peut ainsi être fixée sur le tronçon tubulaire 356 par une goupille transversale 363. L'autre extrémité 366 du barreau 362 est engagée, dans le canal central d'une rotule sphérique 367 positionnée dans le tronçon 352.

En variante le barreau 362 en matériau à mémoire de forme peut être remplacé par toute structure équivalente, par exemple un fil ou un ressort hélicoïdal.

Le tronçon 356 d'extrémité du rotor d'un module 320 donné est de préférence relié au bâti 210 du module aval, tant en translation qu'en rotation, par tous moyens appropriés schématisés sous la référence 380 sur les figures annexées.

Le fonctionnement de ce microcathéter est essentiellement le suivant.

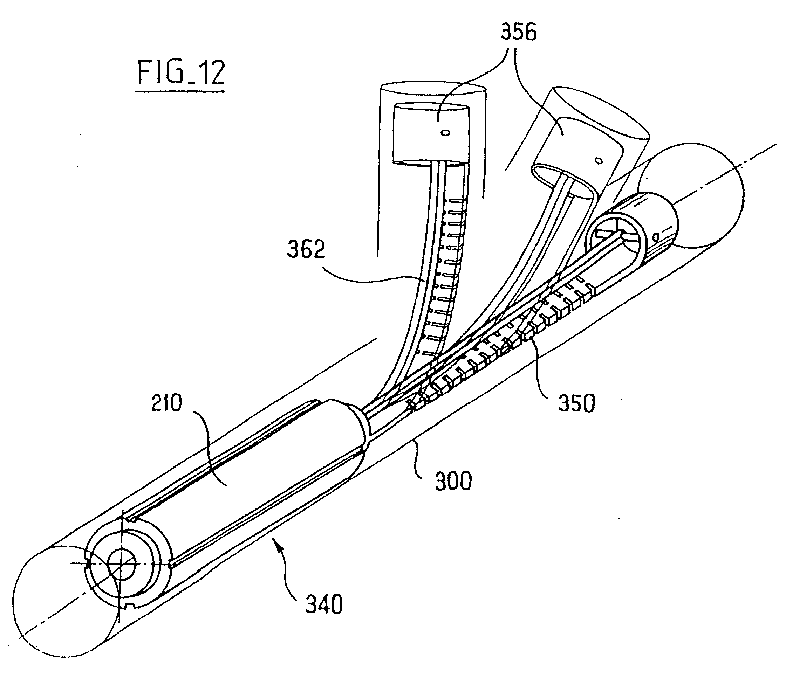

L'actionneur à mémoire de forme 360 permet de contrôler la flexion de l'arbre moteur 350 auquel il est associé, selon un plan de déformation préalablement défini par les paramètres géométriques du rotor élastique 350, comme cela est illustré sur la figure 12.

Le moteur tubulaire 340 est quant à lui adapté pour contrôler le plan de flexion de l'actionneur à mémoire de forme 360.

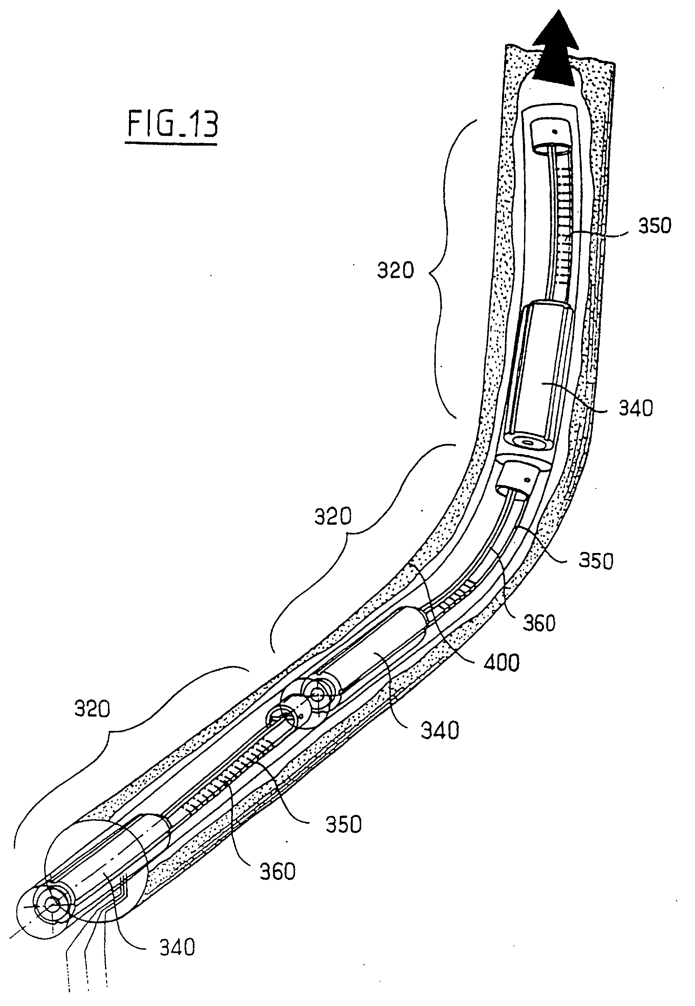

Ce système est schématisé sur la figure 13 dans un vaisseau sanguin 400

Le dispositif global obtenu à l'aide de l'association en série de plusieurs modules 320 élémentaires combinant chacun un actionneur SDA 340 et un actionneur à mémoire de forme 360, permet de maîtriser un grand nombre de configurations bi ou tridimensionnelles (courbures planes avec points d'inflexions multiples, hélicoïdales, ...), malgré une connectique extrêmement simple.

Ainsi les modules 320 conformes à la présente invention combinant un actionneur SDA 340 et un actionneur à mémoire de forme 360 peuvent trouver de nombreuses applications en dehors du microcathéter précité.
[ 1] « Controlled stepwise motion in polysilicon microstructures », T. Akiyama et K. Shono, J. MEMS, Vol. 2 N°3, pp 106-110, 1993 ;
[ 2] « Scratch drive actuator with mechanical links for self-assembly of three dimensional MEMS », T. Akiyama, D. Collard et H. Fujita, J. MEMS, Vol. 26 N°1, pp 14-17, 1997 ;
[ 3] »Self-assembled microactuated XYZ stages for Optical Scanning and Alignment », L. Fan, M. C. Wu, K. D. Choquette et M. H. Crawford, Transducers'97, International Conference on Solid-State Sensors and Actuators, pp319-322, Chicago, 1997.

## Revendications

1. Microactionneur électrostatique à base d'actionneurs élémentaires distribués (110) comprenant chacun une plaque ou poutre flexible (10), munie à une extrémité d'un muret ou coussinet (12) en saillie, dirigé vers un substrat (20) revêtu d'une couche isolante (22) et un générateur (30) adapté pour appliquer des impulsions de tension entre la plaque (10) et le substrat (20), **caractérisé par le fait qu'**il intègre un grand nombre de tels actionneurs élémentaires (110) associés et des moyens (130) pour appliquer individuellement, sur la totalité des actionneurs (110) une précontrainte mécanique extérieure par l'usage de moyens garantissant une superposition des forces motrices générées par les différents actionneurs élémentaires et, d'autre part, transférer à une charge extérieure commune, la résultante des forces motrices issues du comportement collectif de ces mêmes actionneurs élémentaires, les actionneurs élémentaires (110) étant formés dans une feuille support (100) monolithique placée dans un jeu mécanique à l'interface de deux corps solides (200, 210) articulés l'un par rapport à l'autre, de telle sorte que le cadre de la feuille (100) soit en contact avec l'un des corps solides (210), tandis que les actionneurs élémentaires (110) sont en contact avec l'autre corps (200) impliqué dans l'articulation.

2. Microactionneur selon la revendication 1, **caractérisé par le fait que** les moyens adaptés pour appliquer une pression mécanique extérieure sur les actionneurs élémentaires (110) sont formés de moyens (130) aptes à appliquer une tension de polarisation au repos à l'ensemble des actionneurs élémentaires (110).

3. Microactionneur selon l'une des revendications 1 ou 2, **caractérisé par le fait que** la feuille support (100) est flexible et l'un des corps solides (210), est formé d'un bâti, tandis que l'autre corps (200) impliqué dans l'articulation est formé d'un élément mobile.

4. Microactionneur selon l'une des revendications 1 à 3, **caractérisé par le fait que** la feuille (100) support des actionneurs élémentaires est séparée de son substrat avant l'insertion dans le jeu mécanique entre les deux corps solides (200, 210).

5. Microactionneur selon l'une des revendications 1 à 4, **caractérisé par le fait que** chaque plaque (10) formant un actionneur élémentaire est supportée élastiquement dans un cadre (14) ménagé dans la feuille support (100).

6. Microactionneur selon la revendication 5, **caractérisé par le fait que** chaque plaque (10) formant un actionneur élémentaire est supportée par un cadre (14) ménagé dans la feuille (100), par l'intermédiaire de deux barres de suspension parallèles (16).

7. Microactionneur selon l'une des revendications 1 à 6, **caractérisé par le fait que** la plaque ou poutre (10) est formée en polysilicium.

8. Microactionneur selon l'une des revendications 1 à 7, **caractérisé par le fait qu'**il comprend de quelques dizaines à quelques milliers d'actionneurs élémentaires (110).

9. Microactionneur selon l'une des revendications 1 à 8, **caractérisé par le fait que** les actionneurs élémentaires (110) sont réalisés dans une feuille (100) qui possède des barres de flexion (120) adaptées pour assurer une mise en forme de la feuille, lorsque ces barres de flexion (120) sont bloquées sur un substrat (150), tandis que certains au moins des actionneurs élémentaires (110) sont activés.

10. Microactionneur selon l'une des revendications 1 à 9, **caractérisé par le fait qu'**il comprend en outre des moyens (180) formant capteur de force, par exemple capteur de couple, comportant au moins une poutre (182) intégrée à la feuille formant les actionneurs élémentaires et adaptée pour être déformée lors de l'actionnement du système, ladite poutre étant associée à des moyens d'analyse de sa déformation.

11. Microactionneur selon l'une des revendications 1 à 10, **caractérisé par le fait que** les actionneurs élémentaires (110) sont réalisés par usinage chimique de feuilles minces en polysilicium dopé.

12. Microactionneur selon la revendication 9, **caractérisé par le fait que** le substrat (150) comprend des électrodes (160) en regard des barres de flexion (120) et des moyens aptes à appliquer une tension d'excitation entre ces électrodes (160) et les barres de flexion (120) pour plaquer les barres de flexion (120) contre les électrodes (160).

13. Microactionneur selon la revendication 12, **caractérisé par le fait qu'**il comprend plusieurs électrodes (160) discrètes réparties en regard des barres de flexion (120), susceptibles d'être alimentées sélectivement pour contrôler la zone des barres de flexion (120) maintenue sur le substrat (150).

14. Microactionneur selon l'une des revendications 1 à 13, **caractérisé par le fait que** la feuille d'actionneurs élémentaires (100) est placée sur un arbre tubulaire (200).

15. Microactionneur selon l'une des revendications 1 à 14, **caractérisé par le fait qu'**il comporte deux séries d'actionneurs élémentaires (110) possédant des orientations orthogonales, pour former un translateur plan de type X-Y.

16. Microactionneur selon l'une des revendications 1 à 15, **caractérisé par le fait qu'**il constitue un moteur de type verrou associant un degré de liberté de rotation à un degré de liberté de translation.

17. Microactionneur selon l'une des revendications 1 à 16, **caractérisé par le fait qu'**il constitue un moteur de type annulaire.

18. Microactionneur selon la revendication 17, **caractérisé par le fait qu'**il comporte une feuille plane (100) comportant un grand nombre d'actionneurs élémentaires (110), en forme de disque transversal à son axe de rotation 0.

19. Microactionneur selon la revendication 18, **caractérisé par le fait que** la feuille (100) qui constitue un rotor comporte un grand nombre d'actionneurs élémentaires (110) agencés radialement à partir d'un moyeu central (102), les murets (12) de chacune des plaques (10) en forme de secteur de couronne des actionneurs élémentaires, s'étendant le long d'un bord longitudinal de ces plaques, selon un rayon dressé à partir de l'axe de rotation O, et de préférence sur une partie seulement de l'extension radiale de ces secteurs de couronne.

20. Microactionneur selon la revendication 10, **caractérisé par le fait que** chaque poutre (182) possède une extrémité (181) solidaire de la feuille (100), tandis que l'autre extrémité (183) de la poutre (182) est libre par rapport à ladite feuille (100).

21. Microactionneur selon la revendication 20, **caractérisé par le fait que** le capteur (180) comprend trois poutres (182) équiréparties autour de l'axe de rotation O du rotor.

22. Microactionneur selon la revendication 21, **caractérisé par le fait que** les poutres (182) s'étendent dans le sens de rotation à partir de leur extrémité (181) liée à la feuille (100).

23. Microactionneur selon la revendication 21, **caractérisé par le fait que** les poutres (182) sont plaquées sur un support (150) par des forces électrostatiques résultant de l'application d'une tension adéquate appliquée par un générateur (136) entre chaque poutre (182) et des électrodes (152) formées sur le support (150) en regard du trajet de déplacement des poutres (182).

24. Microactionneur selon l'une des revendications 1 à 23, **caractérisé par le fait qu'**il est associé à un actionneur à mémoire de forme (360).

25. Microactionneur selon la revendication 24, **caractérisé par le fait qu'**il comporte au moins un module (320) comprenant un moteur rotatif (340) à base d'actionneurs élémentaires (110) et un barreau de flexion (362) à mémoire de forme lié au rotor (350) du moteur rotatif (340).

26. Microactionneur selon la revendication 25, **caractérisé par le fait qu'**il comprend plusieurs modules (320) en série.

27. Microactionneur selon l'une des revendications 24 à 26, **caractérisé par le fait qu'**il est placé dans un tube souple (310).

28. Microactionneur selon l'une des revendications 25 à 27, **caractérisé par le fait que** le bâti tubulaire (210) du moteur rotatif (340) est muni de gorges longitudinales (212) sur sa périphérie extérieure, pour permettre le passage de fils d'alimentation électrique isolés (214), nécessaires à l'alimentation de modules (320) situés en aval.

29. Microactionneur selon l'une des revendications 25 à 28, **caractérisé par le fait que** le rotor (350) comprend deux tronçons tubulaires (352, 356) d'extrémité reliés entre eux par un bras de liaison longitudinal (354).

30. Microactionneur selon la revendication 29, **caractérisé par le fait que** l'une des extrémités d'un barreau (362) en matériau à mémoire de forme est fixée sur l'un des tronçons du rotor, tandis que l'autre extrémité du barreau (362) est adaptée à l'autre tronçon du rotor, avec liberté de déplacement longitudinal par rapport à celui-ci, pour autoriser une flexion du barreau (362) et du rotor (350).

31. Microactionneur selon la revendication 30, **caractérisé par le fait que** une extrémité (364) du barreau (362) est fixée sur un tronçon tubulaire (356) de rotor par une goupille transversale (363), tandis que l'autre extrémité (366) du barreau (362) est engagée, dans le canal central d'une rotule sphérique (367) positionnée dans un second tronçon (352) de rotor.

32. Microactionneur selon l'une des revendications 26 à 31, **caractérisé par le fait que** une extrémité du rotor d'un module (320) donné est reliée au bâti (210) du module aval, tant en translation qu'en rotation.

33. Microactionneur selon l'une des revendications 1 à 32, **caractérisé par le fait qu'**il constitue un microcathéter (300).

34. Microcathéter tridimensionnel actif comprenant au moins un microactionneur conforme à l'une des revendications 1 à 33.

35. Procédé de fabrication d'un microactionneur conforme à l'une des revendications précitées, **caractérisé par le fait qu'**il met en oeuvre une feuille (100) comportant un grand nombre d'actionneurs élémentaires (110) et qui possède des barres de flexion (120), et qu'il comporte une étape de mise en forme de la feuille par blocage des barres de flexion (120) sur un substrat (150), tandis que certains au moins des actionneurs élémentaires (110) sont activés, pour décoller ladite feuille (100) du substrat.

36. Procédé selon la revendication 35, **caractérisé par le fait qu'**un élément mobile de moteur, tel qu'un rotor (200) est déplacé sous la feuille (100) au fur et à mesure du décollement de celle-ci.

37. Procédé selon la revendication 36, **caractérisé par le fait que** ledit élément moteur (200) est animé d'un mouvement de rotation au cours de son déplacement sous la feuille (100) décollée de sorte que ledit élément moteur (200) vient soutenir la feuille (100) au fur et à mesure de son décollement, sans déplacement relatif entre la surface de l'élément moteur (200) et la feuille (100).

38. Procédé selon l'une des revendications 36 ou 37, **caractérisé par le fait que** une attraction électrostatique est générée entre la feuille (100) et ledit élément moteur (200) lors du transfert de la feuille sur ce dernier.

39. Procédé selon la revendication 38, **caractérisé par le fait que** l'attraction électrostatique est générée par application d'une tension entre la feuille (100) et ledit élément moteur (200).

40. Procédé selon l'une des revendications 35 à 39, **caractérisé par le fait qu'**une attraction électrostatique est maintenue entre la feuille d'actionneurs élémentaires (100) et un élément mobile (200) formant support de celle-ci, tel qu'un rotor, lors de l'introduction de cet élément (200) dans un bâti (210), tandis qu'après mise en place de l'élément (200) dans le bâti on applique une attraction électrostatique entre le bâti (210) et des cadres de la feuille (100) entourant chaque actionneur élémentaire (110) pour immobiliser le corps de la feuille par rapport au bâti (210) tout en permettant aux murets actifs (12) des actionneurs élémentaires de reposer sur ledit élément mobile pour entrainer celui-ci lors de l'excitation des actionneurs élémentaires (110).

## Patentansprüche

1. Elektrostatisches Mikrostellglied basierend auf verteilten Grundstellgliedern (110), die jeweils eine flexible Platte oder einen flexiblen Träger (10) aufweisen, die/der an einem Ende mit einer kleinen Mauer oder einem vorstehenden Auflager (12) versehen ist, die/der auf ein mit einer isolierenden Schicht (22) bedecktes Substrat (20) und einen Generator (30) gerichtet ist, der angepaßt ist, um Spannungspulse zwischen der Platte (10) und dem Substrat (20) anzuwenden, **dadurch gekennzeichnet, daß** es eine große Zahl solcher Grundstellglieder (110) integriert, die mit Mitteln (130) verbunden sind, um individuell auf die Gesamtheit der Stellglieder (110) durch die Verwendung von Mitteln, die eine Überlagerung von Bewegungskräften, die durch die verschiedenen Grundstellglieder erzeugt werden, eine äußere mechanische Vorspannung anzuwenden und, andererseits, die Resultierende dieser Bewegungskräfte, die von dem kollektiven Verhalten dieser selben Grundstellglieder ausgehen, auf eine gemeinsame äußere Last zu übertragen, wobei die Grundstellglieder (110) in einer monolithischen Trägerfolie (100) gebildet sind, die in einem mechanischen Spielraum an der Schnittstelle von zwei zueinander beweglichen festen Körpern (200, 210) derart angeordnet ist, daß der Rahmen der Folie (100) in Kontakt mit dem einen der festen Körper (210) ist, während die Grundstellglieder (110) mit dem anderen, an dem Gelenk beteiligten Körper (200) in Kontakt sind.

2. Mikrostellglied nach Anspruch 1, **dadurch gekennzeichnet, daß** die Mittel zum Anwenden eines äußeren mechanischen Druckes auf die Grundstellglieder (110) aus Mitteln (130) gebildet sind, die geeignet sind, eine Spannung zur Polarisation in der Ruhelage auf das Ensemble der Grundstellglieder (110) anzuwenden.

3. Mikrostellglied nach Anspruch 1 oder 2, **dadurch gekennzeichnet, daß** die Trägerfolie (100) flexibel ist und der eine der festen Körper (210) aus einem Gestell gebildet ist, während der andere, an dem Gelenk beteiligte Körper (200) aus einem beweglichen Element gebildet ist.

4. Mikrostellglied nach einem der Ansprüche 1 bis 3, **dadurch gekennzeichnet, daß** die Trägerfolie (100) der Grundstellglieder vor dem Eindringen in den mechanischen Spielraum zwischen den beiden festen Körpern (200, 210) von ihrem Substrat getrennt wird.

5. Mikrostellglied nach einem der Ansprüche 1 bis 4, **dadurch gekennzeichnet, daß** jede Platte (10), die ein Grundstellglied bildet, elastisch in einem Rahmen (14) gehalten wird, der in der Trägerfolie (100) ausgespart ist.

6. Mikrostellglied nach Anspruch 5, **dadurch gekennzeichnet, daß** jede Platte (10), die ein Grundstellglied bildet, von einem in der Folie (100) ausgesparten Rahmen (14) mittels zweier paralleler Hängestäbe (16) gehalten wird.

7. Mikrostellglied nach einem der Ansprüche 1 bis 6, **dadurch gekennzeichnet, daß** die Platte oder der Träger (10) aus Polysilicium gebildet ist.

8. Mikrostellglied nach einem der Ansprüche 1 bis 7, **dadurch gekennzeichnet, daß** es von einigen Dutzenden bis einigen Tausenden Grundstellglieder (110) umfaßt.

9. Mikrostellglied nach einem der Ansprüche 1 bis 8, **dadurch gekennzeichnet, daß** die Grundstellglieder (110) in einer Folie realisiert sind, die Biegeelemente (120) zum Sichern einer Formgebung der Folie besitzt, wenn diese Biegeelemente (120) auf einem Substrat (150) blockiert sind, während wenigstens einige der Grundstellglieder (110) aktiviert sind.

10. Mikrostellglied nach einem der Ansprüche 1 bis 9, **dadurch gekennzeichnet, daß** es außerdem Mittel (180) umfaßt, die einen Kraftmeßfühler, z.B. einen Momentenmeßfühler, bilden, der wenigstens einen Träger (182) aufweist, der in die die Grundstellglieder bildende Folie integriert ist und angepaßt ist, um während des Betriebs des Systems deformiert zu werden, wobei der Träger mit Mitteln zur Analyse seiner Deformation verbunden ist.

11. Mikrostellglied nach einem der Ansprüche 1 bis 10, **dadurch gekennzeichnet, daß** die Grundstellglieder (110) durch chemische Verarbeitung von dünnen dotierten Polysiliciumfolien hergestellt werden.

12. Mikrostellglied nach Anspruch 9, **dadurch gekennzeichnet, daß** das Substrat (150) Elektroden (160) gegenüber den Biegeelementen (120) und Mittel umfaßt, die geeignet sind, eine Erregerspannung zwischen diesen Elektroden (160) und den Biegeelementen (120) anzuwenden, um die Biegeelemente (120) gegen die Elektroden (160) zu drücken.

13. Mikrostellglied nach Anspruch 12, **dadurch gekennzeichnet, daß** es mehrere einzelne Elektroden (160) umfaßt, die gegenüber den Biegeelementen (120) verteilt sind und empfindlich sind, selektiv gespeist zu werden, um die Zone der Biegeelemente (120), die auf dem Substrat (150) gehalten werden, zu kontrollieren.

14. Mikrostellglied nach einem der Ansprüche 1 bis 13, **dadurch gekennzeichnet, daß** die Folie der Grundstellglieder (100) auf einer Rohrwelle (200) angeordnet ist.

15. Mikrostellglied nach einem der Ansprüche 1 bis 14, **dadurch gekennzeichnet, daß** es zwei Serien von Grundstellgliedern (110) aufweist, die orthogonale Ausrichtungen besitzen, um einen ebenen Translator vom X-Y Typ zu bilden.

16. Mikrostellglied nach einem der Ansprüche 1 bis 15, **dadurch gekennzeichnet, daß** es einen Motor vom Verriegelungstyp darstellt, der einen Rotationsfreiheitsgrad mit einem Translationsfreiheitsgrad verbindet.

17. Mikrostellglied nach einem der Ansprüche 1 bis 16, **dadurch gekennzeichnet, daß** es einen Motor ringförmiger Art darstellt.

18. Mikrostellglied nach Anspruch 17, **dadurch gekennzeichnet, daß** es eine ebene Folie (100) aufweist, die eine große Zahl Grundstellgliedern (110) in Form einer zu ihrer Rotationsachse O transversalen Scheibe aufweist.

19. Mikrostellglied nach Anspruch 18, **dadurch gekennzeichnet, daß** die Folie (100), die einen Rotor darstellt, eine große Zahl Grundstellglieder (110) aufweist, die ausgehend von einer zentralen Nabe (102) radial angeordnet sind, wobei sich die kleinen Mauern (12) jeder Platte (10) in Form eines Sektors eines Kranzes von Grundstellgliedern entlang eines longitudinalen Randes dieser Platten auf einem von der Rotationsachse O ausgehenden Strahl - erstrecken, und zwar bevorzugt nur auf einen Teil der radialen Ausdehnung dieser Kranzsektoren.

20. Mikrostellglied nach Anspruch 10, **dadurch gekennzeichnet, daß** jeder Träger (182) ein mit der Folie (100) verbundenes Ende (181) besitzt, während das andere Ende (183) des Trägers (182) bezüglich der Folie (100) frei ist.

21. Mikrostellglied nach Anspruch 20, **dadurch gekennzeichnet, daß** der Meßfühler (180) drei Träger (182) umfaßt, die gleichmäßig um die Rotationsachse O des Rotors verteilt sind.

22. Mikrostellglied nach Anspruch 21, **dadurch gekennzeichnet, daß** die Träger (182), ausgehend von ihrem mit der Folie (100) verbundenen Ende (181), sich im Rotationssinn erstrecken.

23. Mikrostellglied nach Anspruch 21, **dadurch gekennzeichnet, daß** die Träger (182) auf ein Trägerelement (150) gedrückt werden durch elektrostatische Kräfte, die von der Anwendung einer angemessenen Spannung resultieren, die durch einen Generator (136) zwischen jedem Träger (182) und Elektroden (152) angewendet wird, die gegenüber der Verschiebungsstrecke der Träger (182) auf dem Trägerelement (150) ausgebildet sind.

24. Mikrostellglied nach einem der Ansprüche 1 bis 23, **dadurch gekennzeichnet, daß** es mit einem Stellglied mit Formspeicher (360) verbunden ist.

25. Mikrostellglied nach Anspruch 24, **dadurch gekennzeichnet, daß** es wenigstens ein Modul (320) aufweist, das einen Drehmotor (340) basierend auf Grundstellgliedern (110) und einen Biegestab (362) mit Formspeicher, der mit dem Rotor (350) des Drehmotors (340) verbunden ist, umfaßt.

26. Mikrostellglied nach Anspruch 25, **dadurch gekennzeichnet, daß** es mehrere Module (320) in Serie umfaßt.

27. Mikrostellglied nach einem der Ansprüche 24 bis 26, **dadurch gekennzeichnet, daß** es in einem biegsamen Rohr (310) angeordnet ist.

28. Mikrostellglied nach einem der Ansprüche 25 bis 27, **dadurch gekennzeichnet, daß** das Rohrgestell (210) des Drehmotors (340) mit longitudinalen Vertiefungen (212) in seinem äußeren Bereich versehen ist, um das Durchführen von isolierten elektrischen Versorgungskabeln (214) zu ermöglichen, die zur Versorgung von stromabwärts gelegenen Modulen (320) notwendig sind.

29. Mikrostellglied nach einem der Ansprüche 25 bis 28, **dadurch gekennzeichnet, daß** der Rotor (350) zwei Rohrendstücke (352, 356) umfaßt, die untereinander durch einen longitudinalen Verbindungsarm (354) verbunden sind.

30. Mikrostellglied nach Anspruch 29, **dadurch gekennzeichnet, daß** eines der Enden einer Stange (362) aus Formspeichermaterial auf einem der Abschnitte des Rotors fixiert ist, während das andere Ende der Stange (362) an dem anderen Abschnitt des Rotors mit longitudinaler Verschiebungsfreiheit diesem gegenüber angepaßt ist, um eine Biegung des Stabes (362) und des Rotors (350) zuzulassen.

31. Mikrostellglied nach Anspruch 30, **dadurch gekennzeichnet, daß** ein Ende (364) der Stange (362) auf einem Rohrstück (356) des Rotors durch einen transversalen Stift (363) fixiert ist, während das andere Ende (366) der Stange (362) in den zentralen Kanal eines Kugelgelenks (367) eingesteckt ist, das in einem zweiten Abschnitt (352) des Rotors angeordnet ist.

32. Mikrostellglied nach einem der Ansprüche 26 bis 31, **dadurch gekennzeichnet, daß** ein Ende des Rotors eines gegebenen Moduls (320) mit dem Gestell (210) des stromabwärts gelegenen Moduls sowohl in Translation wie in Rotation verbunden ist.

33. Mikrostellglied nach einem der Ansprüche 1 bis 32, **dadurch gekennzeichnet, daß** es einen Mikrokatheter (300) darstellt.

34. Dreidimensionaler aktiver Mikrokatheter, der wenigstens ein Mikrostellglied gemäß einem der Ansprüche 1 bis 33 umfaßt.

35. Herstellungsverfahren eines Mikrostellgliedes gemäß einem der vorangegangenen Ansprüche, **dadurch gekennzeichnet, daß** es eine Folie (100) verarbeitet, die eine große Zahl Grundstellglieder (110) aufweist und die Biegeelemente (120) besitzt, und daß es einen Aufbereitungsschritt der Folie durch Blockieren der Biegeelemente (120) auf einem Substrat (150) aufweist, während wenigstens einige der Grundstellglieder (110) aktiviert sind, um die Folie (100) vom Substrat abzulösen.

36. Verfahren nach Anspruch 35, **dadurch gekennzeichnet, daß** ein bewegliches Element des Motors, wie ein Rotor (200), in dem Maße des Ablösens der Folie (100) unter diese geschoben wird.

37. Verfahren nach Anspruch 36, **dadurch gekennzeichnet, daß** das Motorelement (200) durch eine Rotationsbewegung im Laufe seines Verschiebens unter die abgelöste Folie (100) derart angeregt wird, daß das Motorelement (200) die Folie (100) in dem Maße seines Ablösens ohne Relativverschiebung zwischen der Oberfläche des Motorelements (200) und der Folie (100) unterstützt.

38. Verfahren nach Anspruch 36 oder 37, **dadurch gekennzeichnet, daß** eine elektrostatische Anziehung zwischen der Folie (100) und dem Motorelement (200) während des Übertragens der Folie auf letzteres generiert wird.

39. Verfahren nach Anspruch 38, **dadurch gekennzeichnet, daß** die elektrostatische Anziehung durch Anwendung einer Spannung zwischen der Folie (100) und dem Motorelement (200) erzeugt wird.

40. Verfahren nach einem der Ansprüche 35 bis 39, **dadurch gekennzeichnet, daß** eine elektrostatische Anziehung zwischen der Folie von Grundstellgliedern (100) und einem beweglichen Element (200), das deren Träger bildet, wie einen Rotor, während der Einführung des Elements (200) in das Gestell (210) aufrechterhalten wird, wohingegen nach dem Anordnen des Elements (200) in dem Gestell eine elektrostatische Anziehung zwischen dem Gestell (210) und Rahmen der Folie (100) angewendet wird, die jedes Grundstellglied (110) umgeben, um den Körper der Folie gegenüber dem Gestell (210) zu fixieren, wobei den aktiven kleinen Mauern (12) der Grundstellglieder es ermöglicht wird, auf dem beweglichen Element aufzuliegen, um es während der Erregung der Grundstellglieder (110) mitzuziehen.

## Claims

1. Electrostatic microactuator based on distributed elementary actuators (110) each comprising a flexible plate or beam (10), furnished at one end with a projecting strut or pad (12), directed towards a substrate (20) covered with an insulating layer (22) and a generator (30) adapted for applying voltage pulses between the plate (10) and the substrate (20), **characterized in that** it integrates a large number of such associated elementary actuators (110) and means (130) for applying individually, to the entirety of actuators (110), an external mechanical prestress by using means guaranteeing a superposition of the driving forces generated by the various elementary actuators and, moreover, transferring to a common external load, the resultant of the driving forces emanating from the collective behaviour of these same elementary actuators, the elementary actuators (110) being formed in a monolithic support sheet (100) placed in a mechanical clearance at the interface of two solid bodies (200, 210) articulated with respect to one another, in such a way that the framework of the sheet (100) is in contact with one of the solid bodies (210), while the elementary actuators (110) are in contact with the other body (200) involved in the articulation.

2. Microactuator according to Claim 1, **characterized in that** the means adapted for applying an external mechanical pressure to the elementary actuators (110) are formed of means (130) able to apply a bias voltage at rest to the set of elementary actuators (110).

3. Microactuator according to either of Claims 1 and 2, **characterized in that** the support sheet (100) is flexible and one of the solid bodies (210) is formed of a bedplate, while the other body (200) involved in the articulation is formed of a movable element.

4. Microactuator according to one of Claims 1 to 3, **characterized in that** the elementary actuator support sheet (100) is separated from its substrate before insertion into the mechanical clearance between the two solid bodies (200, 210).

5. Microactuator according to one of Claims 1 to 4, **characterized in that** each plate (10) forming an elementary actuator is supported elastically in a framework (14) created in the support sheet (100).

6. Microactuator according to Claim 5, **characterized in that** each plate (10) forming an elementary actuator is supported by a framework (14) created in the sheet (100), by way of two parallel suspension bars (16).

7. Microactuator according to one of Claims 1 to 6, **characterized in that** the plate or beam (10) is formed from polysilicon.

8. Microactuator according to one of Claims 1 to 7, **characterized in that** it comprises from a few tens to a few thousand elementary actuators (110).

9. Microactuator according to one of Claims 1 to 8, **characterized in that** the elementary actuators (110) are made in a sheet (100) which possesses flexion bars (120) adapted for ensuring shaping of the sheet, when these flexion bars (120) are locked on a substrate (150), while some at least of the elementary actuators (110) are activated.

10. Microactuator according to one of Claims 1 to 9, **characterized in that** it furthermore comprises means (180) forming a force sensor, for example a torque sensor, comprising at least one beam (182) integrated into the sheet forming the elementary actuators and adapted to be deformed upon actuation of the system, the said beam being associated with means for analysing its deformation.

11. Microactuator according to one of Claims 1 to 10, **characterized in that** the elementary actuators (110) are made by chemical machining of thin sheets of doped polysilicon.

12. Microactuator according to Claim 9, **characterized in that** the substrate (150) comprises electrodes (160) facing the flexion bars (120) and means able to apply an excitation voltage between these electrodes (160) and the flexion bars (120) so as to push the flexion bars (120) flat against the electrodes (160).

13. Microactuator according to Claim 12, **characterized in that** it comprises several discrete electrodes (160) distributed facing the flexion bars (120), capable of being energized selectively so as to control the zone of the flexion bars (120) which is held on the substrate (150).

14. Microactuator according to one of Claims 1 to 13, **characterized in that** the elementary actuator sheet (100) is placed on a tubular shaft (200).

15. Microactuator according to one of Claims 1 to 14, **characterized in that** it comprises two series of elementary actuators (110) possessing orthogonal orientations, to form an X-Y type plane translator.

16. Microactuator according to one of Claims 1 to 15, **characterized in that** it constitutes a latch type motor associating a rotational degree of freedom with a translational degree of freedom.

17. Microactuator according to one of Claims 1 to 16, **characterized in that** it constitutes an annular type motor.

18. Microactuator according to Claim 17, **characterized in that** it comprises a plane sheet (100) comprising a large number of elementary actuators (110), in the shape of a disc transverse to its axis of rotation 0.

19. Microactuator according to Claim 18, **characterized in that** the sheet (100) which constitutes a rotor comprises a large number of elementary actuators (110) arranged radially from a central hub (102), the struts (12) of each of the plates (10) in the shape of a ring sector of the elementary actuators, extending along a longitudinal edge of these plates, along a radius pitched from the axis of rotation O and preferably over only a part of the radial extension of these ring sectors.

20. Microactuator according to Claim 10, **characterized in that** each beam (182) possesses an end (181) secured to the sheet (100), while the other end (183) of the beam (182) is free with respect to said sheet (100).

21. Microactuator according to Claim 20, **characterized in that** the sensor (180) comprises three beams (182) equidistributed about the axis of rotation O of the rotor.

22. Microactuator according to Claim 21, **characterized in that** the beams (182) extend in the direction of rotation from their end (181) linked to the sheet (100).

23. Microactuator according to Claim 21, **characterized in that** the beams (182) are pushed flat onto the support (150) by electrostatic forces resulting from the application of a suitable voltage applied by a generator (136) between each beam (182) and electrodes (152) formed on the support (150) facing the displacement path of the beams (182).

24. Microactuator according to one of Claims 1 to 23, **characterized in that** it is associated with a shape memory actuator (360).

25. Microactuator according to Claim 24, **characterized in that** it comprises at least one module (320) comprising an elementary actuator-based rotary motor (340) and a shape memory flexion pin (362) linked to the rotor (350) of the rotary motor (340).

26. Microactuator according to Claim 25, **characterized in that** it comprises several modules (320) in series.

27. Microactuator according to one of Claims 24 to 26, **characterized in that** it is placed in a flexible tube (310).

28. Microactuator according to one of Claims 25 to 27, **characterized in that** the tubular bedplate (210) of the rotary motor (340) is furnished with longitudinal grooves (212) over its outer periphery, so as to allow the passage of insulated electrical supply wires (214) required for energizing modules (320) situated downstream.

29. Microactuator according to one of Claims 25 to 28, **characterized in that** the rotor (350) comprises two tubular end spans (352, 356) joined together by a longitudinal linking arm (354).

30. Microactuator according to Claim 29, **characterized in that** one of the ends of a pin (362) made of shape memory material is fixed to one of the spans of the rotor, while the other end of the pin (362) is fitted to the other span of the rotor, with freedom of longitudinal displacement with respect to the latter, so as to permit flexion of the pin (362) and of the rotor (350).

31. Microactuator according to Claim 30, **characterized in that** one end (364) of the pin (362) is fixed to a rotor tubular span (356) by a transverse stud (363), while the other end (366) of the pin (362) is engaged in the central channel of a ball joint (367) positioned in a second rotor span (352).

32. Microactuator according to one of Claims 26 to 31, **characterized in that** one end of the rotor of a given module (320) is joined to the bedplate (210) of the downstream module, both translationally and rotationally.

33. Microactuator according to one of Claims 1 to 32, **characterized in that** it constitutes a microcatheter (300).

34. Active three-dimensional microcatheter comprising at least one microactuator in accordance with one of Claims 1 to 33.

35. Process for fabricating a microactuator in accordance with one of the aforesaid claims, **characterized in that** it implements a sheet (100) comprising a large number of elementary actuators (110) and which possesses flexion bars (120), and that it comprises a step of shaping the sheet by locking the flexion bars (120) on a substrate (150), while some at least of the elementary actuators (110) are activated, so as to detach the said sheet (100) from the substrate.

36. Process according to Claim 35, **characterized in that** a movable motor element, such as a rotor (200) is displaced under the sheet (100) as the latter is detached.

37. Process according to Claim 36, **characterized in that** the said motor element (200) is accorded a rotational motion in the course of its displacement under the detached sheet (100) so that the said motor element (200) will brace the sheet (100) as it detaches, without relative displacement between the surface of the motor element (200) and the sheet (100).

38. Process according to either of Claims 36 and 37, **characterized in that** an electrostatic attraction is generated between the sheet (100) and the said motor element (200) while transferring the sheet onto the latter.

39. Process according to Claim 38, **characterized in that** the electrostatic attraction is generated by applying a voltage between the sheet (100) and the said motor element (200).

40. Process according to one of Claims 35 to 39, **characterized in that** an electrostatic attraction is maintained between the elementary actuator sheet (100) and a movable element (200) forming a support for the sheet, such as a rotor, upon introduction of this element (200) into a bedplate (210), while after placing the element (200) in the bedplate, an electrostatic attraction is applied between the bedplate (210) and frameworks of the sheet (100) surrounding each elementary actuator (110) so as to immobilize the body of the sheet with respect to the bedplate (210) while allowing the active struts (12) of the elementary actuators to rest on the said movable element so as to drive the latter upon excitation of the elementary actuators (110).
